# EUROPÄISCHE PATENTANMELDUNG

(11) **EP 2 648 120 A1**
(43) Veröffentlichungstag der Anmeldung: **09.10.2013**
(21) Anmeldenummer: 12162892.9
(22) Anmeldetag: 02.04.2012
(51) Int. Cl.: G06F 19/00

(54) **Gerät und Verfahren zum Anzeigen einer Information**

(71) Anmelder: mySugr GmbH, 1050 Wien (AT)
(72) Erfinder: Stangl, Gerald, A-5020 Salzburg (AT); Debong, Carl-Fredrik, A-1070 Wien (AT); Westermann, Frank, D-10407 Berlin (DE); Forisch, Michael, A-1080 Wien (AT)

(57) **Zusammenfassung**

Verfahren zum Wiedergeben einer Information, insbesondere einer Werbeinformation, mit Hilfe eines Gerätes, wobei das Verfahren die folgenden Schritte aufweist:
Prüfen, ob Therapiedaten ein positives Therapieereignis repräsentieren, und Ansteuern einer visuellen und/oder akustischen Wiedergabestufe des Geräts zwecks Wiedergabe von besagter Information, wenn ein Ergebnis der Prüfung besagtes positives Therapieereignis anzeigt.

## Beschreibung

### TECHNISCHES FELD

Die Erfindung betrifft ein Verfahren zum Wiedergeben einer Information mit Hilfe eines Gerätes.

Die Erfindung betrifft weiterhin ein Gerät zum Wiedergeben einer Information.

Die Erfindung betrifft einen Server zum Bereitstellen von Daten.

Die Erfindung betrifft weiterhin eine Verwendung eines Gerätes zur Wiedergabe einer Information.

Die Erfindung betrifft weiterhin ein Computerprogrammprodukt.

Die Erfindung betrifft weiterhin ein Verfahren zum Verrechnen einer Wiedergabe von Information.

### HINTERGRUND

Ein eingangs erwähntes Gerät ist beispielsweise in Form eines Smartphone, nachfolgend kurz Mobiltelefon genannt, eines Tablett-PC oder auch anderer Computer wie z.B. als iPad ® von Apple ® bekannt. Solche Geräte weisen eine Anzeigeeinheit zum Visualisieren von Daten und / oder Information auf. Auf ein solches Mobiltelefon können verschiedenste Programme, auch Applikationen genannt - letztendlich Computerprogrammprodukte - geladen und dort mit Hilfe eines Prozessors ausgeführt werden. Die Applikationen stellen unterschiedlichste Funktionalitäten bereit. Wird ein solches Programmprodukt auf dem Gerät ausgeführt bzw. abgearbeitet, werden Daten, die der Applikation zugeordnet sind, verarbeitet und z.B. Hard- und / oder Softwareschnittstellen des Geräts genutzt, um Information, die durch die Daten repräsentiert ist, mit Hilfe der Anzeigeeinheit zu visualisieren. Dieser Vorgang entspricht dem eingangs im zweiten Absatz angeführten Verfahren bzw. der eingangs im dritten Absatz angeführten Verwendung. Eine solche Applikation kann auch zur benutzerorientierten Protokollierung von Aktivitäten aber auch zur Bereitstellung bzw. Visualisierung von Werbenachrichten an Benutzer des Geräts eingesetzt werden.

Herkömmliche Werbung basiert auf dem Konzept einen möglichst hohen Werbedruck zu erzeugen durch eine möglichst häufigen Wiederholung der Werbeinformation, also der Frequenz, um die Nachricht der Werbung, z.B. die beworbene Marke, gut im Gedächtnis eines Benutzers zu verankern. Diese Art der Bereitstellung einer Werbeinformation kann jedoch auf Seiten des Benutzers durch eine werbliche Überreizung zu einer Irritation führen. Der Nutzer nimmt bewusst oder unterbewusst die Werbebotschaft nicht mehr wahr. Die Erfindung hat sich die Aufgabe gestellt, ein verbessertes Verfahren, ein verbessertes Gerät, einen verbesserten Server, eine neuartige Verwendung, sowie ein neuartiges Computerprogrammprodukt und ein Verfahren zum Verrechnen einer Wiedergabe von Information anzugeben, so dass das eingangs erörterte Problem vermieden ist.

### ZUSAMMENFASSUNG DER ERFINDUNG

Diese Aufgabe wird durch ein Verfahren zum Wiedergeben einer Information gemäß Anspruch 1, ein Gerät gemäß Anspruch 9, durch einen Server gemäß Anspruch 11, ein System gemäß Anspruch 13, eine Verwendung gemäß Anspruch 14, sowie ein Computerprogrammprodukt und ein Verfahren zum Verrechnen einer Wiedergabe einer Information gemäß Anspruch 15 gelöst.

Der Gegenstand der Erfindung ist daher ein Verfahren zum Wiedergeben einer Information, insbesondere einer Werbeinformation, mit Hilfe eines Gerätes, wobei das Verfahren die folgenden Schritte aufweist: Prüfen, ob Therapiedaten ein positives Therapieereignis repräsentieren, und
Ansteuern einer visuellen und/oder akustischen Wiedergabestufe des Geräts zwecks Wiedergabe von besagter Information, wenn ein Ergebnis der Prüfung besagtes positives Therapieereignis anzeigt.

Zudem ist der Gegenstand der Erfindung ein Gerät aufweisend eine Wiedergabestufe zum visuellen und/oder akustischen Wiedergeben einer Information, insbesondere einer Werbeinformation, und eine erste Datenverarbeitungsstufe zum Ansteuern der Wiedergabestufe zwecks Wiedergeben von besagter Information, wobei besagtes Ansteuern dann erfolgt, wenn als Folge einer Prüfung, ob Therapiedaten ein positives Therapieereignis repräsentieren, ein Ergebnis der Prüfung besagtes positives Therapieereignis anzeigt.

Zudem ist der Gegenstand der Erfindung ein Server zum Bereitstellen von Daten aufweisend eine zweite Kommunikationsstufe zum Empfangen von Therapiedaten von einem Gerät und zum Kommunizieren von Definitionsdaten an das Gerät, wobei die Definitionsdaten eine Regel repräsentieren, welche Regel das Vorliegen eines positiven Therapieereignisses definiert, und eine zweite Datenverarbeitungsstufe zum Generieren besagter Definitionsdaten durch Auswerten der Therapiedaten, bevorzugt unter Berücksichtigung eines Profils eines Benutzers des Geräts.

Zudem ist der Gegenstand der Erfindung ein System aufweisend zumindest ein erfindungsgemäßes Gerät und einen erfindungsgemäßen Server.

Zudem ist der Gegenstand der Erfindung eine Verwendung eines Gerätes zur Wiedergabe von Information, insbesondere einer Werbeinformation, wobei besagte Information dann wiedergegeben wird, wenn als Folge einer Prüfung, ob Therapiedaten ein positives Therapieereignis repräsentieren, ein Ergebnis der Prüfung besagtes positives Therapieereignis anzeigt.

Zudem ist der Gegenstand der Erfindung ein Computerprogrammprodukt, welches in ein programmierbares Gerät, insbesondere ein mobiles Gerät, ladbar ist, mit Programmcode-Abschnitten, um die Schritte des erfindungsgemäßen Verfahrens zum Wiedergeben einer Information durchzuführen, wenn das Programm in dem Gerät ausgeführt wird.

Zudem ist der Gegenstand der Erfindung ein Verfahren zum Verrechnen einer Wiedergabe von Information, wobei das Verfahren die folgenden Verfahrensschritte aufweist, nämlich Generieren von Verrechnungsdaten, wobei die Verrechnungsdaten Geldeinheiten repräsentieren und in Abhängigkeit davon generiert werden, dass bei einem Gerät, insbesondere einem mobilen Gerät eine Information, insbesondere eine Werbeinformation, wiedergebbar ist, wenn ein positives Therapieerlebnis eintritt.

Die Therapiedaten liegen in digitaler Form vor und die Prüfung erfolgt automatisch. Die Prüfung kann zeitlich versetzt zum Zeitpunkt des Auftretens eines Therapieereignisses oder unmittelbar nachfolgend dem Eintreten des Therapieereignisses, z.B. als Folge des Eingebens, Erfassens oder Vorliegens von neuen bzw. aktualisierten oder aktuellen Therapiedaten, die das Therapieereignis repräsentieren, gestartet werden oder durch die Eingabe von Therapiedaten durch ein anderes Community-Mitglied um ein Community-Event auszulösen.

Das Gerät kann ein mobiles - insbesondere programmierbares - Gerät, wie z.B. ein Smartphone sein, auf dem eine Applikation als das Computerprogrammprodukt, das z.B. von eine "application store" über das Internet ladbar ist, ausgeführt wird. Das Gerät kann jedoch auch ein Computer, wie z.B. ein Laptop oder PC sein, auf dem eine Web-Applikation als das Computerprogrammprodukt ausgeführt wird. Ebenso kann ein Computer oder ein anderes mobiles Gerät z.B. mit Hilfe eines Webbrowsers eine Website zur Verfügung stellen, welche die Funktionalität des erfindungsgemäßen Verfahrens bereitstellt. Das Gerät kann jedoch auch eine fix verdrahtete Logikschaltung aufweisen, die ein erfindungsgemäßes Verfahren bereitstellt.

Auch wenn in der nachfolgenden Erörterung der Erfindung auf den Spezialbereich der Diabeteserkrankung detailliert eingegangen ist, sei an dieser Stelle bereist erwähnt, dass die Erfindung im Zusammenhang mit jeder chronischen Erkrankung zur Anwendung kommen kann. Die Erfindung eignet sich also im weitesten Sinne für die Unterstützung jeder Therapie einer chronischen Erkrankung, deren Therapie ein hohes Maß an Selbstdisziplin konsequentes Handeln des Patenten erfordert. Im Fall von anderen chronischen Erkrankungen als der Diabetes, wie z.B. Bluthochdruck, Epilepsie, Koronare Herzkrankheit, Asthma, Parkinson, Brustkrebs, Endometriose, Multiple Sklerose, Apoplex, Alkoholismus, Demenz, Epilepsie, Gicht, Rheuma, Colitis ulcerosa, Morbus Crohn, ändern sich lediglich die Parametrisierungen der Therapie und gegebenenfalls auch die damit einhergehende Definitionen eines positiven Therapieereignisses.

Mit den erfindungsgemäßen Maßnahmen geht der Vorteil einher, dass eine zeitlich ungünstige Inszenierung einer Information zuverlässig vermieden ist. In Abkehr von der herkömmlichen Strategie zur Präsentation von Werbung, die ein möglichst häufiges Anzeigen der Werbeinformation vorsieht, ermöglicht die Erfindung die Inszenierung einer werbetreibenden Marke in einem positiven Therapiekontext. Die Erfindung vermeidet also die Bereitstellung von Information, insbesondere die Schaltung von Werbung für chronisch kranke Menschen, zum Beispiel Diabetiker, zu einem Zeitpunkt, zu dem man nicht genau weiß, in welchem Gemütszustand der Empfänger der Werbung ist. Es wird somit die Gefahr vermieden, dass die Marke des Werbetreibenden zum falschen Zeitpunkt wahrgenommen wird. Ein falscher Zeitpunkt würde mit Sicherheit dann vorliegen, wenn der Empfänger der Werbung in einem schlechten Gemütszustand ist. Insbesondere bei Diabetikern besteht die Wahrscheinlichkeit, dass sich diese bei einem ungewöhnlich hohen oder niedrigem Zuckerwert nicht wohl fühlen. So haben Diabetiker mit einer schlechten Blutzuckereinstellung oft ein hohes Risiko, depressiv zu werden. Bei Vorliegen eines schlechten Gemütszustands, also eines schlechten Therapiekontexts, wenn zum Beispiel der Test des Blutzuckerwerts einen schlechten Blutzuckerwert anzeigt, empfindet ein Diabetiker Werbung als störend und projiziert seinen schlechten Gemütszustand auf die werbetreibende Marke, was zu einer negativen Markenwahrnehmung führt. Der Sinn der Werbung, nämlich eine positive Markenwahrnehmung zu erzielen, würde in einem solchen Zustand des Empfängers der Werbung nicht erreicht und Kosten, die für das Schalten der Werbung beim werbenden Unternehmen entstehen, als wirtschaftlich nicht erwünscht und ungerechtfertigt klassifiziert werden. Die Erfindung folgt dem Grundgedanken, dass die Wahrscheinlichkeit, dass ein chronisch kranker Patient dank eines Therapieerfolges in einem positiven Gemütszustand ist, relativ hoch ist. In diesem positiven Gemütszustand werden präsentierte Informationen, wie z.B. motivierende Worte aber auch Werbeinformationen, wie beispielsweise eine visuell präsentierte Marke, im Bewusstsein eines Benutzer des Geräts in einem positiven Kontext verankert. Dies wird dadurch erreicht, dass dem Patienten bzw. Benutzer des Gerätes die Werbung nur im Zusammenhang mit dem Vorliegen eines positiven Therapieereignisses angezeigt wird. Damit ist erreicht, dass der Benutzer des Gerätes unterbewusst die Marke mit einem positiven Therapieerfolg bzw. Therapieereignis verbindet und sich somit eine positive Markenwahrnehmung etabliert. Dies wiederum erhöht die Bereitschaft von Unternehmern, Werbeinformation gegen Bezahlung Benutzern des Geräts zukommen zu lassen, weil letztendlich die positive Markenwahrnehmung das Ziel der werbetreibenden Unternehmen ist. Diese Bereitschaft ermöglicht ein neues Geschäftsmodell zu etablieren, auf das nachfolgend noch eingegangen ist, wobei die Erfindung die technischen Rahmenbedingungen dafür etabliert.

Weitere, besonders vorteilhafte Ausgestaltungen und Weiterbildungen der Erfindung ergeben sich aus den abhängigen Ansprüchen sowie der nachfolgenden Beschreibung. Dabei kann das Gerät, der Server, das System und auch die Verwendung entsprechend den abhängigen Ansprüchen zum Verfahren weitergebildet sein. Vorteile, die im Zusammenhang mit Merkmalen der einen Kategorie erörtert werden, treffen analog auch für jene der anderen Kategorie zu. Gemäß einem Aspekt der Erfindung repräsentieren die Therapiedaten - oder in anderen Worten Gesundheitsdaten - zumindest ein Therapieereignis ausgewählt aus der folgenden Gruppe: Blutdruckwerte, körperliche Aktivität, Nahrung (z.B. Menge und Art), Blutdruck, Gewicht, Cholesterin, allgemeine Blutwerte, Einnahme von Tabletten, Spritzen von Insulin, keine Kopfschmerzen, keine Bauchschmerzen, Community-Vergleich, Verkettung der zuvor genannten.

Ein Blutzuckerwert kann beispielsweise von einem Diabetiker selbst bestimmt werden. Dies kann zum Beispiel mit Hilfe eines Teststreifens erfolgen, auf denen ein Blutstropfen aufgebracht wird, bevor er in ein Blutzuckerwert-Bestimmungsgerät eingeführt wird. Der mit Hilfe des Bestimmungsgeräts ermittelte Blutzuckerwert wird manuell über eine Tastatur oder einen Touchscreen des Gerätes eingegeben. Der Blutzucker kann jedoch auch auf automatische Weise übertragen werden und z.B. über eine Funkverbindung mit einem Blutzuckerwert-Bestimmungsgerät in das Gerät übernommen werden. Es hat sich zudem auch als vorteilhaft erwiesen, wenn der Blutzuckerwert nicht isoliert, sondern im Zusammenhang mit jenem Kontext protokolliert wird, in dem er auftritt. Ein solcher Kontext kann beispielsweise die körperliche Aktivität, die aufgenommene Nahrung, ein genommenes Medikament wie z.B. Insulin oder eine Serie bzw. eine zeitliche Abfolge solcher Therapieereignisse darstellen.

Die körperliche Aktivität kann beispielsweise auch manuell von dem Patienten über die Tastatur eingegeben werden. Sie kann jedoch auch auf automatische Weise in dem Gerät aufgezeichnet werden, wie beispielsweise mit einem elektronischen Schrittzähler oder mit Hilfe von einem GPS-Empfänger (in dem Gerät integriert oder separat ausgeführt).

Ein Beispiel für eine Beziehung bzw. Verkettung der vorgenannten Therapieereignisse kann eine Verkettung von Bewegung und Blutzuckerwert sein, die sich in der Form "erfasster Blutzuckerwert zwei Stunden nach einem Tennisspiel" manifestiert.

Jedes der Therapieereignisse wird digital mit Hilfe von zugeordneten Daten erfasst und z.B. für Zwecke der Auswertung und / oder Analyse und / oder Entscheidung gespeichert. Der Blutzuckerwert wird durch Blutzuckerwertdaten, die Aktivität durch Aktivitätsdaten, die Nahrung durch Nahrungsdaten repräsentiert. Verkettungsdaten repräsentieren die jeweils zutreffende Verkettung der Ereignisse. Ein Community-Vergleich wird durch Vergleichsdaten repräsentiert, die ein Ergebnis des Vergleichs der Therapiedaten des Benutzers des Gerätes mit jenen der Community (Gemeinschaft, Gruppe von Menschen, die ein gemeinsames Ziel verfolgen, gemeinsame Interessen pflegen - hier die Therapie einer chronischen Erkrankung, insbesondere von Diabetes) angeben.

Gemäß einem weiteren Aspekt der Erfindung ist das Vorliegen eines positiven Therapieereignisses über zumindest eine Regel berücksichtigend zumindest ein Element ausgewählt aus der folgenden Gruppe definiert: Häufigkeit des Therapieereignisses; Anzahl der erfassten Therapieereignisse; Vielfalt der erfassten Therapieereignisse; Erreichen, Unter- oder Überschreiten eines für das jeweilige Therapieereignisses relevanten Zielwerts oder eines Zielwertbereiches; Therapieereignis in Bezug zu anderen Nutzern.

Wenn beispielsweise der Blutzuckerwert als Indikator für ein positives Therapieereignis herangezogen wird, können beispielhaft folgende Situationen angeführt werden: Ein Diabetiker hat in der letzten Woche fünfzig Mal einen Zuckerwert in die Applikation eingegeben. Er hat keine Zuckerwerte unter 200 mg/dl (Milligramm /Deziliter) gehabt. Obwohl es nach offiziellen Standards der Weltgesundheitsorganisation (WHO) bzw. ärztlichen Standards keine gute Therapie ist, kann es für den Diabetiker persönlich ein positives Therapieereignis sein, einen einzigen Blutzuckerwert von unter 150 mg/dl zu haben. Bei Vorliegen dieses positiven Therapieereignisses könnte also die wiederzugebende Information auf dem Bildschirm des Gerätes die Botschaft enthalten: "Super, Du hast zum ersten Mal seit einer Woche einen Zuckerwert unter 150 mg/dl gehabt! Weiter so!". Die zugrundeliegende Regel berücksichtigt also hinsichtlich des Blutzuckerwerts einen zu unterschreitenden Zielwert von 150 mg/dl als relevanten Wert und hinsichtlich der Häufigkeit des Therapieereignisses ein einziges Auftreten des Zielwerts in einem Zeitintervall von einer Woche.

Die Definition eines positiven Therapieevents bzw. -Therapieereignisses hängt also unabhängig der vorgegebenen Richtlinien, z.B. die der WHO, von der individuellen Qualität der Diabetestherapie ab.

Die Höhe des Blutzuckerwertes, der als ein positives Therapieereignis zu klassifizieren ist, kann von historischen Blutzuckerwertdaten des Benutzers abhängen. Sind keine solchen Blutzuckerwertdaten vorhanden, so kann z.B. auf offizielle Daten der WHO zurückgegriffen werden.

Ein weiteres Szenario in Bezug auf den Blutzuckerwert als positives Therapieereignis könnte wie folgt gestaltet sein: Ein Diabetiker hat in der letzten Woche fünfzig Mal einen Zuckerwert in die Applikation auf dem Gerät eingetragen. Er hatte nie einen Blutzuckerwert über 150mg/dl. Beim fünfzigsten Eintrag infolge, bei dem ein Blutzuckerwert von unter 150mg/dl protokolliert wird, erfolgt die Wiedergabe der Information: "Wow! Herzlichen Glückwunsch, Du hast fünfzig Mal infolge einen Blutzuckerwert unter 150mg/dl gehabt!". Bei der zugrundeliegenden Regel wurde als relevanter Wert, den es in ununterbrochener Folge zu unterschreiten gilt, ein Blutzuckerwerte von 150mg/dl definiert. Zudem wurde hinsichtlich der Häufigkeit dieses Therapieereignisses ein zu erreichender Wert von 50 definiert.

Es kann jedoch auch die Art Nahrung als ein positives Therapieereignis eingestuft werden, wobei in so einem Fall folgendes Szenario eintreten könnte: Der Diabetiker protokolliert in seinem Gerät, dass er einen Apfel gegessen hat. Daraufhin gibt das Gerät die Information wieder: "Super! Ein Apfel ist gesund!". Die zugrundeliegende Regel definiert also, was im Sinne der für den jeweiligen Benutzer festgelegten Therapieempfehlungen als eine gesunde Nahrung eingestuft wird, und setzt als zeitlichen Maßstab die Dauer eines Tages fest.

Für den Fall, dass Community-Daten für einen Community-Vergleiche zur Verfügung stehen, könnte sich bei einem Vergleich mit anderen Patienten herausstellen, dass sich der betreffende Patient besser ernährt hat als alle anderen in der Community registrierten Patienten. In einem solchen Fall könnte die Information folgende Nachricht aufweisen: "Super! Du hast Dich besser ernährt als alle anderen Diabetiker in Wien!". Die zugrundeliegende Regel fordert also den Vergleich des Blutzuckerwerts jedes Mitglieds der Community mit jenen des Benutzers des Geräts, eingeschränkt auf den zeitlichen Horizont eines Tages.

Ein solcher Community-Vergleich könnte in Bezug auf körperliche Aktivität auch feststellen, dass der betreffende Benutzer im Sinne der für ihn individuell entwickelten Therapieempfehlungen die richtige körperliche Aktivität, im richtigen Umfang und mit der richtigen Belastung durchgeführt hat, und alle anderen Mitglieder der Community sich undiszipliniert verhalten haben. In einem solchen Fall könnte die wiederzugebende Information folgenden Inhalt aufweisen: "Super, Du hast heute auf ideale Weise Sport betrieben und Du bist heute der aktivste Diabetiker Wiens!". Die zugrundeliegende Regel berücksichtigt bzw. bewertet die für den betroffenen Benutzer als richtig definierte Aktivität hinsichtlich Art, Umfang (Anzahl der Übungen oder Wiederholungen und / oder Zeitdauer) und körperliche Anstrengung, die durch manuelle Eingabe in das gerät oder auf automatische Weise z.B. mit Hilfe eines Pulsmessers oder per GPS erfasst wurde, und setzt diese Bewertung in Relation zu Bewertungen anderer Benutzer der Community. Zudem definiert die Regel als zeitlichen Horizont einen Tag oder einem anderen frei zu definierenden Zeitraum.

Selbst nach einer längeren Nichtbenutzung der Applikation zur Protokollierung von Therapieereignissen kann die erstmalige Wiederbenutzung der Applikation als positives Therapieereignis eingestuft werden und eine mögliche Information folgenden Inhalt aufweisen: "Welcome back! Wir freuen uns, dass Du Dich nach so langer Zeit wieder um Deine Diabetes kümmerst und einen Blutzuckerwert eingibst!". Die zugrundeliegende Regel definiert als Maßeinheit für das Feststellen einer Nichtbenutzung eine Zeitspanne von z.B. einer Woche und ein einziges erfasstes Therapieereignis nach Ablauf dieser Zeitspanne als ein positives Therapieereignis.

Ebenso können in Abhängigkeit von der jeweiligen Situation die Häufigkeit oder die Vielfalt der erfassten Therapieereignisse zu einem positiven Therapieereignis führen, was in einer entsprechenden Regel manifestieret wird.

Die Festlegung einer solchen Regel oder mehrerer zu berücksichtigender Regeln und das Eintreten des Therapieereignisses für einen Benutzer des Geräts kann grundsätzlich auch auf dem Gerät selbst erfolgen. Es hat sich jedoch als besonders vorteilhaft erwiesen, wenn die Therapiedaten an den Server übermittelt und von dem Server Definitionsdaten empfangen und gespeichert werden, welche Definitionsdaten besagte Regel repräsentieren, wobei die Regel mit Hilfe des Servers durch Auswertung der übermittelten Therapiedaten definiert wurde. Das Gerät kann also autonom bei Vorliegen einer Datenverbindung zwischen dem Gerät und einem Server die Therapiedaten an den Server übermitteln. Mit Hilfe des Servers wird besagte Regel durch Auswertung der Therapiedaten definiert und die Regel repräsentierende Definitionsdaten gespeichert. Sofort nach Generierung der Definitionsdaten oder zeitverzögert dazu, wenn z.B. eine Datenverbindung mit dem Gerät abgebrochen wurde, werden die Definitionsdaten an das Gerät übertragen, wo sie für besagte Prüfung gespeichert werden. Somit können aufwändigere Dataminingaktivitäten - insbesondere in Bezug auf Community-Vergleiche oder statistische Auswertungen der Therapiedaten - auf den leistungsfähigen Server ausgelagert werden. Zudem stehen die so erhaltenen und in dem Server nunmehr gespeicherten Therapiedaten eines Benutzers auch für Community-Vergleiche mit anderen Nutzern (Community-Mitgliedern) zur Verfügung.

Durch das Speichern der Definitionsdaten in dem Gerät ist auf Seiten des Gerätes keine Internet- bzw. Datenverbindung mit dem Server mehr nötig, um zu prüfen ob ein positives Therapieereignis vorliegt. Es kann also das Vorliegen eines positiven Therapieereignisses autonom auf dem Gerät durchgeführt werden.

Mit Hilfe des Geräts, genauer der auf dem Gerät ausgeführten Applikation, werden bei einer erstmaligen Verwendung möglichst alle verfügbaren Nutzerdaten und, soweit verfügbar auch initiale Therapiedaten lokal erfasst und gespeichert. Diese Nutzer- und Therapiedaten werden bei Vorhandensein einer Internet- bzw. Datenverbindung zu dem Server übertragen, wo der Benutzer registriert wird und ein ihm zugeordnetes Profil erstellt wird, das in Form von Profildaten auf dem Server gespeichert wird. In der Anfangsphase der Benutzung des Geräts kann eine auf dem Gerät zur Anwendung kommende Regel zum Erkennen eines positiven Therapieereignisses beispielsweise fix vorgegeben sind. Dies wird dann der Fall sein, wenn zu dem Benutzer noch keine ausreichenden Informationen und / oder Therapiedaten existieren. Bei jeder Erfassung eines Therapieereignisses, das der Benutzer mit Hilfe des Geräts protokolliert, werden die aktuellen Therapiedaten lokal in dem Gerät gespeichert und sobald eine Internet- bzw. Datenverbindung existiert auf den Server übertragen. Sobald Therapiedaten eingegeben werden, wird vorsorglich bereits auf dem Gerät unter Berücksichtigung der jeweils verfügbaren Regel(n) die Existenz eines positiven Therapieereignisses geprüft, und zwar selbst dann, wenn momentan keine Internet- bzw. Datenverbindung existiert. Es wird also immer auf das zuletzt gültige Regelwerk, das in dem Gerät gespeichert ist, zurückgegriffen, um aktualisierte Therapiedaten zu bewerten.

Sobald jedoch eine relevante Anzahl an Therapieereignissen in Form von Therapiedaten auf dem Server vorhanden ist, kann serverseitig bei der Generierung der Regel(n) auch von den historisch erfassten Therapiedaten eines Benutzers gebrauch gemacht werden. Die historisch erfassten Therapiedaten sind dem Profil des Benutzers zugeordnet. Neben den historisch gespeicherten Therapiedaten kann das Profil auch persönliche Informationen wie Alter, Gewicht, Größe usw. aber auch auf den Benutzer abgestimmte Therapieempfehlungen eines Arztes enthalten. Alle diese Informationen können zusätzlich zu den Therapiedaten in die Definition der jeweiligen Regel(n) einfließen. Demgemäß kann laut einem weiteren Aspekt der Erfindung das positive Therapieereignis von einem Profil eines Benutzers abhängig gemacht werden. Dies ist deshalb von Vorteil, weil ein positives Therapieereignis z.B. bei der Diabetestherapie nicht oder nur sehr grob generalisiert werden kann. Die Diabetestherapie ist facettenreich und die Qualität der Therapie individuell unterschiedlich. Üblicherweise bewegt sich jeder Diabetiker in einem anderen Bereich des Blutzuckerwerts. Was für den einen Diabetiker bereits ein Erfolg im Sinne eines positiven Therapieerlebnisses ist, kann für einen anderen Diabetiker ein alltägliches Erlebnis sein. Es ist daher von besonderem Vorteil, wenn die Bereitstellung der Information, insbesondere der Werbeinformation, individuell auf die Qualität bzw. die Einhaltung der Therapie abgestellt ist, was sich in individualisierten Regeln manifestiert, die auf alle Facetten der Therapie Rücksicht nehmen.

Gemäß einem weiteren Aspekt der Erfindung kann das Prüfen, ob ein positives Therapieereignis vorliegt, mit Hilfe einer geräteseitig, ersten Datenverarbeitungsstufe oder einer serverseitigen, zweiten Datenverarbeitungsstufe erfolgen. Erfolgt die Prüfung serverseitig, so können größere Datenmengen und aufwändigere Datenanalyseverfahren zur Anwendung kommen. Es kann bei dieser Variante jedoch vorkommen, dass beim Fehlen einer Datenverbindung mit dem Gerät das Auftreten eines positiven Therapieereignisses nicht unmittelbar an das Gerät kommuniziert werden kann und somit die Wiedergabe der Information verzögert erfolgt. Diese Situation ist vermieden, wenn, wie bereits erwähnt, die Prüfung auf Vorliegen eines positiven Therapieereignisses lokal, also geräteseitig in dem Gerät erfolgt. Mit dieser Maßnahme ist in jedem Fall sichergestellt, dass unmittelbar bei Auftreten des positiven Therapieereignisses eine entsprechende Information abgegeben werden kann, die sich bei dem Benutzer in einem positiven Kontext verankert.

Besagte Werbeinformation wird mit Hilfe von Werbedaten repräsentiert, die mit Hilfe eines Servers an das Gerät übermittelt bzw. übertragen wurden, wo die Werbedaten für besagtes visuelle und/oder akustische Wiedergabe gespeichert werden. Die Werbedaten werden lokal auf dem Gerät abgespeichert und werden beim Eintreffen eines positiven Therapieereignisses angezeigt. Das Aussehen und/oder der Wortlaut und/oder der akustische Inhalt der Werbeinformation werden serverseitig definiert. Sobald eine Internet- bzw. Datenverbindung mit dem Gerät existiert, wird das Gerät mit den entsprechenden Werbedaten versorgt. Somit ist sichergestellt, dass zeitgerecht, insbesondere auch anpasst an den Kontext des Eintretens des positiven Therapieereignisses Werbeinformation in dem Gerät zur Verfügung steht. Diese kann auch jederzeit auf einfache Weise upgedatet werden und so relativ rasch an die Bedürfnisse des werbenden Unternehmens angepasst werden. In Bezug auf das werbende Unternehmen besteht zudem der Vorteil, dass diese Unternehmen keinen direkten Zugang zu den Geräten benötigen, weil die gesamte technische und administrative Logistik der Bereitstellung und Übermittlung von Werbeinformation an den Betreiber des Servers übergeben wird. In dem Gerät sind die jeweiligen Werbeinformationen in Form von Werbedaten lokal gespeichert und können jederzeit beim Auftreten des positiven Therapieereignisses wiedergegeben werden.

Die Werbeinformation kann beispielsweise lediglich eine Abbildung einer Marke sein. Zudem kann die Werbeinformation auch lediglich aus einer Anzahl von Wörtern oder Sätzen bestehen, die einen lobenden oder motivierenden Inhalt haben. Als besonders vorteilhaft hat es sich jedoch erwiesen, wenn die Werbeinformation eine personalisierte Werbeinformation ist, die einen Informationsanteil ausgewählt aus der folgenden Gruppe aufweist: beworbene Marke, betroffener Benutzer, positives Therapieereignis. Bei einer Kombination aller oder einzelner Teile dieser Informationsanteile ist der Vorteil erhalten, dass dem Benutzer ein unmittelbarer Zusammenhang zwischen dem soeben eingetretenen positiven Therapieereignis, das letztendlich nur ihn selbst betrifft, und der beworbenen Marke kommuniziert wird. Dies verstärkt die Verankerung der Marke im positiven Kontext.

Auch kann die Art und Weise des festgestellten positiven Therapieereignisses einen Einfluss auf die Art und Weise der Wiedergabe der Werbeinformation haben. So kann beispielsweise in Abhängigkeit von dem soeben protokollierten Therapieereignis, welches als Auslöser für das Erkennen eines positiven Therapieereignisses identifiziert wird, eine unterschiedliche Farbe oder eine unterschiedliche Größe oder eine unterschiedliche Position auf dem Bildschirm des Gerätes gewählt werden. Auch kann die akustische Wiedergabe der Werbeinformation bzw. ihres akustischen Inhaltes davon abhängen, welche angewendete Regel zur Identifikation eines positiven Therapieereignisses führte. Es lassen sich somit in ihrem Intensitäts- oder Motivationsgrad unterschiedlich starke Werbeimpulse beim Eintreten des positiven Therapieereignisses setzten.

In dem erfindungsgemäßen System können ein Server oder ein Serververbund Aufgabe der Bereitstellung von Werbeinformation und Regeln, sowie die Verwaltung von Benutzerprofilen wahrnehmen. Die Anzahl der Geräte kann letztendlich der Anzahl der Benutzer entsprechen, die eine Applikation auf ihren Geräten ausführen, welche das erfindungsgemäße Verfahren etabliert. Daten, die in dem System existieren sind digitaler Natur, sie werden mit Hilfe der jeweiligen Speichermedien gespeichert, mit Hilfe der jeweiligen Verarbeitungsstufen digital verarbeitet und als Datensignale zwischen den verschiedenen strukturellen Elementen kommuniziert.

Mit Hilfe des Servers, kann ein Verfahren zum Verrechnen einer Wiedergabe von Information, insbesondere einer Werbeinformation, ausgeführt werden, wobei das Verfahren die folgenden Verfahrensschritte aufweist, nämlich Generieren von Verrechnungsdaten, wobei die Verrechnungsdaten Geldeinheiten repräsentieren und in Abhängigkeit davon generiert werden, dass bei einem Gerät, insbesondere einem mobilen Gerät die Information wiedergebbar ist, wenn ein positives Therapieerlebnis eintritt, oder besagte Information wiedergegeben wurde, nachdem ein positives Therapieereignis eintrat.

Besagtes System ermöglicht folglich eine Geschäftsmethode, bei der z.B. jene Benutzer des Geräts, die in einer Zeitperiode aktiv sind, dem werbenden Unternehmen pauschal verrechnet werden. Es wird also serverseitig geprüft, ob ein Benutzer über sein Gerät angemeldet ist und entsprechende Anmeldungsdaten als Grundlage für die Verrechnung der Möglichkeit, Werbung im positiven Therapiekontext zu schalten, herangezogen. Es ist auch möglich, individuell oder zu dem zuvor beschriebenen Geschäftsmethode zusätzlich für jede tatsächlich erfolgte Werbeeinschaltung, also pro Wiedergabe der Information, eine Gebühr in Rechnung zu stellen. Dabei wird die erfolgte Werbeeischaltung im Gerät in Form von Protokolldaten protokolliert, die dann an den Server übertragen werden und dort zwecks Generierung der Verrechnungsdaten mit der serverseitigen zweiten Datenverarbeitungsstufe ausgewertet werden..

Da die Einhaltung der Therapieanweisungen des Arztes einen wichtigen Beitrag zur Leistungsfähigkeit, zum Gesundheitszustand und letztendlich auch zum Wohlbefinden eines chronisch Kranken, z.B. eines Diabetikers selbst liefert, kann gemäß einem weiteren Geschäftsmodell auch vorgesehen sein, dass der Arbeitgeber oder auch Krankenkassen als Interessent bzw. Nutznießer von positiven Therapieereignissen - letztendlich einer erfolgreichen Therapie - an der Nutzung der Erfindung durch Diabetiker interessiert sind. Die jeweilige Anzahl der in einer Abrechnungsperiode aktiven Benutzer kann dann auf Grundlage einer serverseitigen Auswertung der Anmeldungsdaten oder der Protokolldaten dem jeweiligen Interessenten gemäß einem Verrechnungsschlüssel in Rechnung gestellt werden. Die Zuordnung der Benutzer zu einem Interessenten kann z.B. unter Benutzung einer durch den Interessenten bereitgestellten E-Mail-Adresse (z.B. Benutzer A gehört zu Interessent OMV - Zuordnung über seine E-Mail Adresse BenutzerA@OMV.at; Benutzer B gehört zu Telekom - Zuordnung über seine E-Mail Adresse BenutzerB@Telekom.at) oder einer anderen eindeutigen Zuordnungsinformation erfolgen.

### FIGURENKURZBESCHREIBUNG

Die Erfindung wird im Folgenden unter Hinweis auf die beigefügten Figuren anhand von Ausführungsbeispielen noch einmal näher erläutert, auf welches die Erfindung jedoch nicht beschränkt ist. Dabei sind in den verschiedenen Figuren gleiche Komponenten mit identischen Bezugszeichen versehen. Es zeigen auf schematische Weise:
- Fig. 1: ein erfindungsgemäßes System mit einem mobilen Gerät in Form eines Blockschaltbildes;
- Fig. 2: ein erfindungsgemäßes Verfahren in Form eines Flussdiagramms;
- Fig. 3: einen ersten Screenshot einer beispielhaften Eingabe eines Therapieereignisses;
- Fig. 4: einen zweiten Screenshot nach erfolgter Eingabe des Therapieereignisses;
- Fig. 5: einen dritten Screenshot enthaltend eine beispielhafte Werbeinformation;
- Fig. 6: einen vierten Screenshot enthaltend eine weitere beispielhafte Werbeinformation.

### BESCHREIBUNG DER AUSFÜHRUNGSBEISPIELE

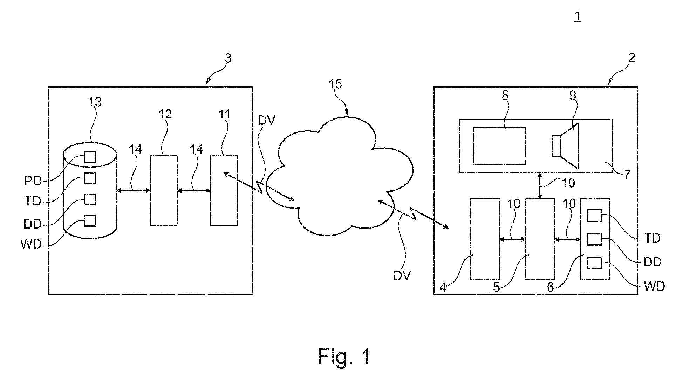

In der Figur 1 ist ein System 1 aufweisend ein einziges mobiles Gerät 2 in Form eines Smartphone und einen Server 3 dargestellt.

Das mobile Gerät 2 weist eine erste Kommunikationsstufe 4, eine erste Datenverarbeitungsstufe 5, eine erste Speicherstufe 6 und eine Wiedergabestufe 7 auf. Die Wiedergabestufe 7 weist zur visuellen Wiedergabe von Information und zur Eingabe von Therapieereignissen ein Display in Form eines Touchscreen 8 auf. Die Wiedergabestufe 7 weist zudem zur akustischen Wiedergabe von Information einen Lautsprecher 9 auf. Die Stufen 4-7 sind mit Hilfe von Hardware und / oder Software (z.B. Mikroprozessor, Peripherie-, Treiber- und / oder Schnittstellenbausteine, Speicherbausteine, sowie Firmware und / oder Betriebssystemroutinen und / oder Softwareroutinen einer Applikation.) realisiert, wobei die entsprechenden Hardwarekomponenten zur Daten- und Signalkommunikation miteinander mit einem ersten Systembus 10 verbunden sind.

Die erste Datenverarbeitungsstufe 5 dient zum Ansteuern der Wiedergabestufe 7 zwecks Wiedergabe von besagter Information. Die erste Kommunikationsstufe 5 dient zum Datenaustausch über eine Datenverbindung DV mit dem Server 3. Dabei wird die ersten Kommunikationsstufe 4 mit Hilfe der ersten Datenverarbeitungsstufe 5 angesteuert, um Therapiedaten TD an den Server 3 zu übertragen und von dem Server 3 Werbedaten WD und Definitionsdaten DD zu empfangen.

Der Server 3 weist eine zweite Kommunikationsstufe 11, eine zweite Datenverarbeitungsstufe 12 und eine zweite Speicherstufe 13 auf. Die Stufen 11-13 sind mit Hilfe von Hardware und / oder Software (z.B. Mikroprozessor, Peripherie-, Treiber- und / oder Schnittstellenbausteine, Speicherbausteine, sowie Firmware und / oder Betriebssystemroutinen und / oder Softwareroutinen einer Applikation) realisiert, wobei die entsprechenden Hartwarekomponenten zur Daten- und Signalkommunikation miteinander mit einem zweiten Systembus 14 verbunden sind.

Die zweite Kommunikationsstufe 11 dient zum Empfangen von Therapiedaten TD von dem mobilen Gerät 2 und zum Kommunizieren von Definitionsdaten DD an das mobile Gerät 3. Die zweite Datenverarbeitungsstufe 12 dient zum Definieren besagter Definitionsdaten DD durch Auswerten der empfangenen Therapiedaten TD bevorzugt unter Berücksichtigung eines Profils eines Benutzers des mobilen Gerätes 2, das in der dritten Speicherstufe 13 in Form von Profildaten PD gespeichert ist. Die zweite Datenverarbeitungsstufe 12 dient zudem zum Bereitstellen einer Werbeinformation repräsentiert in Form von Werbedaten WD als die von dem mobilen Gerät 2 wiederzugebende Information. Die Werbedaten WD werden zusammen mit den Definitionsdaten DD oder separat von diesen mit Hilfe der zweiten Kommunikationsstufe 11 an das Gerät 2 übertragen.

Zwischen dem Server 3 und dem mobilen Gerät 2 ist eine Wolke 15 angedeutet, die eine Internet- bzw. Datenverbindung DV repräsentiert. Diese Datenverbindung kann mit Hilfe von Leitungssignalen und/oder Funksignalen realisiert sein.

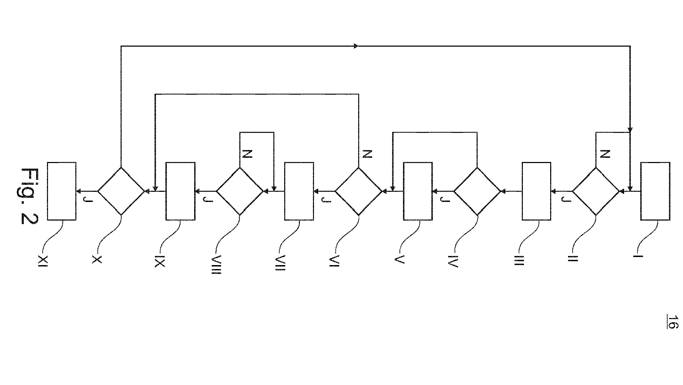

Mit dem mobilen Gerät 2 wird ein Verfahren 16 zum Wiedergeben der Werbeinformation abgearbeitet, das in der Figur 2 visualisiert ist. Dazu wird mit Hilfe des Prozessors (nicht explizit dargestellt) des Geräts 2 ein Programm oder in anderen Worten eine Applikation, kurz App genannt, abgearbeitet, so dass die nachfolgend beschriebene Funktionalität bereitgestellt wird. Das Verfahren 16 beginnt in einem Block I, bei dem z.B. die Applikation gestartet bzw. aktiviert wird.

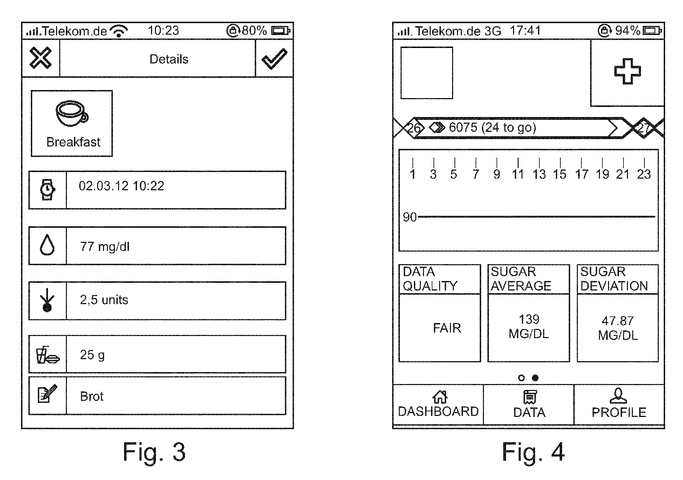

In einem Block II wird geprüft, ob ein Therapieereignis von einem Benutzer des Gerätes 2 mit Hilfe des Touchscreen 8 protokolliert wurde, was zu neuen Therapiedaten TD führt. Beispielhaft ist in der Figur 3 ein Bildschirminhalt visualisiert, der die Elemente des zu protokollierenden Therapieereignisses anzeigt. Zu sehen ist, dass es sich um das Therapieereignis "Frühstück" handelt, das zu einem bestimmten Datum und einer Uhrzeit erfasst wird. Es wird ein Blutzuckerwert von 77 mg/dl eingegeben. Weiterhin wird als ein weiteres Element der Wert 2,5 Units (Insulineinheiten) eingegeben. Zudem wird angegeben, dass es sich um 25 g Kohlenhydrate in Form von Brot handelt. Die Eingabe des Blutzuckerwertes erfolgt daher im Kontext: Typ der Mahlzeit, Menge und Art der aufgenommenen Nahrung, Uhrzeit.

Nachdem alle Daten eingegeben sind, betätigt der Benutzer ein Bestätigungsfeld am rechten oberen Rand des Bildschirms. In der Figur 4 ist der Bildschirminhalt des Touchscreens 8 nach erfolgter Therapiedateneingabe zu sehen, wobei die verschiedensten für den Diabetiker interessanten Informationen angezeigt werden, worauf im Detail jedoch nicht eingegangen ist. Das in der Figur 2 visualisierte Verfahren wird in dem Block III fortgesetzt, wo die soeben erfassten Therapiedaten TD des soeben besprochenen Therapieereignisses in der ersten Speicherstufe 6 gespeichert werden.

Als Nächstes wird bei einem Block IV geprüft ob eine Daten- bzw. Internet-Verbindung vorhanden ist. Wenn dies der Fall ist, werden die Therapiedaten TD bei einem Block V über die Datenverbindung DV an den Server 3 übermittelt und dort in der zweiten Speicherstufe 13 zur späteren Verwendung bzw. Auswertung gespeichert, wonach das Verfahren 16 bei einem Block VI fortgesetzt wird.

Wenn in dem Block IV festgestellt wird, dass keine Daten- bzw. Internet-Verbindung existiert, wird der Block V übersprungen und das Verfahren 16 bei dem Block VI fortgesetzt. Dort erfolgt mit Hilfe der ersten Datenverarbeitungsstufe 5 ein Prüfen, ob die digital gespeicherte Therapiedaten TD ein positives Therapieereignis repräsentieren oder nicht. Im vorliegenden Fall sind in der ersten Speicherstufe 6 Definitionsdaten DD gespeichert. Die Definitionsdaten DD werden von dort mit Hilfe der ersten Datenverarbeitungsstufe 5 ausgelesen und die repräsentierte Regel angewendet. Im vorliegenden Fall besagt die Regel, dass die Bedingung erfüllt sein muss, dass der Blutzuckerwert nach Aufnahme des Frühstücks niedriger als 120 mg/dl sein soll.

Ist die Bedingung nicht erfüllt, verzweigt das Verfahren 16 von dem Block VI zu dem Block X, bei dem getestet wird, ob das Verfahren 16 beendet werden soll, was einem Beenden bzw. Unterbrechen der Applikation gleichkommt. Verläuft dieser Test positiv, verzweigt das Verfahren 16 von dem Block X zu dem Block XI, bei dem die Applikation abgebrochen wird.

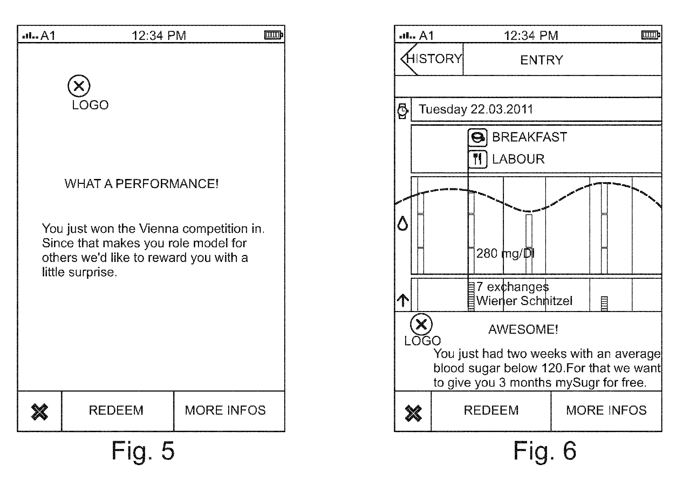

Wird bei dem Block VI festgestellt, dass ein positives Therapieereignis vorliegt, also besagte Bedingung erfüllt ist, wird zu dem Block VII verzweigt, bei dem mit Hilfe der ersten Datenverarbeitungseinrichtung 5 Werbedaten WD aus der ersten Speicherstufe 6 ausgelesen und gegebenenfalls zur Wiedergabe mit Hilfe der Wiedergabeeinrichtung 7 aufbereitet werden. Die gegebenenfalls aufbereiteten Werbedaten WD werden sodann von der ersten Datenverarbeitungsstufe 5 an die Wiedergabeeinrichtung 7 abgegeben, was einem Ansteuern der Wiedergabestufe 7 des Geräts 2 zwecks Wiedergabe von besagter Information entspricht, sobald ein Ergebnis der Prüfung besagtes positives Therapieereignis anzeigt. In der Figur 5 und in der Figur 6 sind zwei mögliche Visualisierungen der Werbeinformation dargestellt, wobei in der Figur 5 der gesamte zur Verfügung stehende Bildschirmbereich des Touchscreen 8 und in der Figur 6 lediglich ein Teilbereich des Touchscreen 8 für die Wiedergabe der Werbeinformation benutzt wird.

In der Figur 2 wird das Verfahren 16 nachfolgend an den Block VII in dem Block VIII fortgesetzt, wo während der Visualisierung der Werbeinformation auf eine Benutzerreaktion gewartet wird. Für eine Benutzerreaktion stehen im vorliegenden Fall drei Möglichkeiten, so wie in den Figuren 5 bis 6 am untersten Rand des Touchscreen 8 abgebildet, zur Verfügung. Die erste Reaktionsmöglichkeit ist Abbrechen durch Betätigen der Schaltfläche im links unten lokalisierten Bereich des Touchscreens 8 visualisiert als "X". Eine weitere, zweite Möglichkeit der Benutzerreaktion besteht darin, eine angebotene Belohnung einzulösen - visualisiert als "REDEEM". Letztendlich besteht auch die Möglichkeit, dass der Benutzer mehr Informationen erhält bzw. anfordert, wenn er eine Schaltfläche im rechts unten lokalisierten Bereich des Touchscreens 8 berührt - visualisiert als "MORE INFO". Sobald mit Hilfe der ersten Datenverarbeitungsstufe 5 eine Benutzerreaktion festgestellt wurde, verzweigt das Verfahren 16 zu dem Block IX, bei dem die entsprechende Benutzerreaktion ausgewertet und entsprechende Aktivitäten gesetzt werden, auf die an dieser Stelle nicht näher eingegangen ist. Das Warten auf eine Benutzerreaktion kann jedoch auch durch Zeitablauf abgerochen werden und zur Fortsetzung des Verfahrens 16 im Block X führen, was jedoch nicht visualisiert ist.

Nach dem Block IX wird das Verfahren 16 bei dem Block X fortgesetzt, bei dem - wie bereits erwähnt - geprüft wird, ob die Applikation oder in anderen Worten das laufende Programm bei dem Block XI abzubrechen oder zu unterbrechen ist. Ist dies nicht der Fall, verzweigt das Verfahren 16 zurück zu dem Block II.

Es wird abschließend noch einmal darauf hingewiesen, dass es sich bei den vorangehend detailliert beschriebenen Figuren nur um Ausführungsbeispiele handelt, welche vom Fachmann in verschiedenster Weise modifiziert werden können, ohne den Bereich der Erfindung zu verlassen. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Auch können individuell offenbarte Merkmale mit anderen Merkmalen kombiniert werden und Proportionen der dargestellten Merkmale variieren, ohne dass von dem Konzept der Erfindung abgewichen wird.

## Patentansprüche

1. Verfahren (16) zum Wiedergeben einer Information, insbesondere einer Werbeinformation, mit Hilfe eines Gerätes (2), insbesondere eines mobilen Gerät (2), wobei das Verfahren (16) die folgenden Schritte aufweist:
Prüfen, ob Therapiedaten (TD) ein positives Therapieereignis repräsentieren, und
Ansteuern einer visuellen und/oder akustischen Wiedergabestufe (7) des Geräts (2) zwecks Wiedergabe von besagter Information, wenn ein Ergebnis der Prüfung besagtes positives Therapieereignis anzeigt.

2. Verfahren (16) nach Anspruch 1, wobei die Therapiedaten (TD) zumindest ein Therapieereignis ausgewählt aus der folgenden Gruppe repräsentieren: Blutzuckerwert; körperliche Aktivität; Nahrung; Blutdruck; Gewicht; Cholesterin; allgemeine Blutwerte; Einnahme von Tabletten; Spritzen von Insulin; keine Kopfschmerzen; keine Bauchschmerzen; Community Vergleiche; Verkettung der zuvor genannten.

3. Verfahren (16) nach einem der vorangehenden Ansprüche, wobei das Vorliegen eines positiven Therapieereignisses über zumindest eine Regel berücksichtigend zumindest ein Element ausgewählt aus der folgenden Gruppe definiert ist: Häufigkeit des Therapieereignisses; Anzahl der erfassten Therapieereignisse; Vielfalt der erfassten Therapieereignisse; Erreichen, Unter oder Überschreiten eines für das jeweilige Therapieereignis relevanten Zielwerts oder eines Zielwertbereiches; Therapieereignis in Bezug zu anderen Nutzern.

4. Verfahren (16) nach Anspruch 3, wobei das Gerät (2)
- die Therapiedaten (TD) an einen Server (3) übermittelt und
- von dem Server (3) Definitionsdaten (DD) empfängt und speichert, welche Definitionsdaten (DD) besagte Regel repräsentieren, wobei die Regel mit Hilfe des Servers (3) durch Auswertung der übermittelten Therapiedaten (TD) definiert wurde.

5. Verfahren (16) nach einem der vorangehenden Ansprüche, wobei das positive Therapieereignis von einem Profil eines Benutzers abhängig ist.

6. Verfahren (16) nach einem der vorangehenden Ansprüche, wobei das Prüfen, ob ein positives Therapieereignis vorliegt, mit Hilfe einer geräteseitigen ersten Datenverarbeitungsstufe (5) oder einer serverseitigen zweiten Datenverarbeitungsstufe (12) erfolgt.

7. Verfahren (16) nach einem der vorangehenden Ansprüche, wobei die Information eine Werbeinformation ist, die mit Hilfe von Werbedaten (WD) repräsentiert wird, die mit Hilfe eines Servers (3) generiert und von dem Server (3) an das Gerät (2) übermittelt wurden, wo die Werbedaten (WD) für besagte Wiedergabe gespeichert werden.

8. Verfahren (16) nach einem der vorangehenden Ansprüche, wobei die Werbeinformation eine personalisierte Werbeinformation ist, die einen Informationsanteil ausgewählt aus der folgenden Gruppe aufweist: beworbene Marke; betroffener Benutzer; positive Therapieereignis; Ort an dem sich der Nutzer befindet.

9. Gerät (2), insbesondere mobiles Gerät (2), aufweisend
- eine Wiedergabestufe (7) zum visuellen und/oder akustischen Wiedergeben einer Information, insbesondere einer Werbeinformation, und
- eine erste Datenverarbeitungsstufe (5) zum Ansteuern der Wiedergabestufe (7) zwecks Wiedergeben von besagter Information, wobei besagtes Ansteuern dann erfolgt, wenn als Folge einer Prüfung, ob Therapiedaten (TD) ein positives Therapieereignis repräsentieren, ein Ergebnis der Prüfung besagtes positives Therapieereignis anzeigt.

10. Gerät (2) nach Anspruch 9 aufweisend eine erste Kommunikationsstufe (4) zum Datenaustausch mit einem Server (3), wobei die erste Datenverarbeitungsstufe (5) ausgebildet ist zum Ansteuern der ersten Kommunikationsstufe (4) zwecks:
- Übertragung der Therapiedaten (TD) an den Server (3) und / oder
- Empfang von Werbedaten (WD) von dem Server (3), welche Werbedaten (WD) besagte Information als Werbeinformation repräsentieren, und / oder
- Empfang von Definitionsdaten (DD) von dem Server (3), welche Definitionsdaten (DD) eine Regel repräsentieren, welche Regel das Vorliegen eines positiven Therapieereignisses definiert.

11. Server (3) zum Bereitstellen von Daten aufweisend
eine zweite Kommunikationsstufe (11) zum Empfangen von Therapiedaten (TD) von einem Gerät (2), insbesondere einem mobilen Gerät(2), und zum Kommunizieren von Definitionsdaten (DD) an das Gerät (2), wobei die Definitionsdaten (DD) eine Regel repräsentieren, welche Regel das Vorleigen eines positiven Therapieereignisses definiert, und
eine zweite Datenverarbeitungsstufe (12) zum Generieren besagter Definitionsdaten (DD) durch Auswerten der Therapiedaten (TD), bevorzugt unter Berücksichtigung eines Profils eines Benutzers des Geräts (2).

12. Server (3) nach Anspruch 11, wobei die zweite Datenverarbeitungsstufe (12) zur Bereitstellung einer Werbeinformation repräsentiert in Form von Werbedaten (WD) als eine von dem Gerät (2) wiederzugebende Information ausgebildet ist, welche Werbedaten (WD) zusammen mit den Definitionsdaten (DD) oder separat davon mit Hilfe der zweiten Kommunikationsstufe (11) an das Gerät (2) übertragbar sind.

13. System (1) aufweisend zumindest ein Gerät (2), insbesondere ein mobiles Gerät (2), nach einem der Ansprüche 9 bis 10 und einen Server (3) nach einem der Ansprüche 11 bis 12.

14. Verwendung eines Gerätes (2), insbesondere eines mobilen Geräts (2) zur visuellen und / oder akustischen Wiedergabe von Information, insbesondere einer Werbeinformation, wobei besagte Information dann wiedergegeben wird, wenn als Folge einer Prüfung, ob Therapiedaten (TD) ein positives Therapieereignis repräsentieren, ein Ergebnis der Prüfung besagtes positives Therapieereignis anzeigt.

15. Verfahren zum Verrechnen einer Wiedergabe von Information, insbesondere einer Werbeinformation, wobei das Verfahren die folgenden Verfahrensschritte aufweist, nämlich Generieren von Verrechnungsdaten, wobei die Verrechnungsdaten Geldeinheiten repräsentieren und in Abhängigkeit davon generiert werden, dass bei einem Gerät, insbesondere einem mobilen Gerät, die Information wiedergebbar ist, wenn ein positives Therapieerlebnis eintritt, oder besagte Information wiedergegeben wurde, nachdem ein positives Therapieereignis eintrat.
